# EUROPEAN PATENT SPECIFICATION

(11) **EP 3 409 105 B1**
(45) Date of publication and mention of the grant of the patent: **29.10.2025**
(21) Application number: 17813402.9
(22) Date of filing: 15.06.2017
(51) Int. Cl.: C12N 15/82, C12N 9/12, C12N 15/54, C12N 15/29, A01H 3/04, A01G 7/06, A01H 3/00, A01N 63/00, C07K 14/415

(54) **METHOD FOR IMPROVING SALT TOLERANCE OF PLANT**
VERFAHREN ZUR VERBESSERUNG DER SALZTOLERANZ EINER PFLANZE
PROCÉDÉ PERMETTANT D'AMÉLIORER LA TOLÉRANCE AU SEL D'UNE PLANTE

(30) Priority: 17.06.2016 JP 2016121235; 13.12.2016 JP 2016241469; 25.04.2017 JP 2017086654; 19.05.2017 JP 2017100286
(43) Date of publication of application: 05.12.2018
(73) Proprietor: Sekisui Chemical Co., Ltd., Osaka-shi, Osaka 530-8565 (JP)
(72) Inventor: ONO, Seigo, Tsukuba-shi Ibaraki 300-4292 (JP); SHIMATANI, Zenpei, Tsukuba-shi Ibaraki 300-4292 (JP)
(74) Representative: Ter Meer Steinmeister & Partner
(86) International application number: PCT/JP2017/022187
(87) International publication number: WO 2017/217508

(56) References cited:
- WO-A2-2006/053246
- WO-A2-2006/079045
- JP-A- 2011 500 081
- JP-A- 2015 149 954
- YOURA HWANG ET AL: "Cell wall-associated ROOT HAIR SPECIFIC 10, a proline-rich receptor-like kinase, is a negative modulator of Arabidopsis root hair growth", JOURNAL OF EXPERIMENTAL BOTANY, vol. 67, no. 6, 16 February 2016 (2016-02-16), GB, pages 2007 - 2022, XP055447937, ISSN: 0022-0957, DOI: 10.1093/jxb/erw031
- YOSR Z HAFFANI ET AL: "Altered expression of PERK receptor kinases in Arabidopsis leads to changes in growth and floral organ formation", PLANT SIGNALING AND BEHAVIOUR, vol. 1, no. 5, 1 September 2006 (2006-09-01), pages 251 - 260, XP055585062
- S.-K. WON ET AL: "cis-Element- and Transcriptome-Based Screening of Root Hair-Specific Genes and Their Functional Characterization in Arabidopsis", PLANT PHYSIOLOGY, vol. 150, no. 3, 15 May 2009 (2009-05-15), Rockville, Md, USA, pages 1459 - 1473, XP055585035, ISSN: 0032-0889, DOI: 10.1104/pp.109.140905
- LING BAI ET AL: "Plasma membrane-associated proline-rich extensin-like receptor kinase 4, a novel regulator of Ca 2+ signalling, is required for abscisic acid responses in Arabidopsis thaliana", THE PLANT JOURNAL, vol. 60, no. 2, 1 October 2009 (2009-10-01), GB, pages 314 - 327, XP055585054, ISSN: 0960-7412, DOI: 10.1111/j.1365-313X.2009.03956.x
- HWANG ET AL.: "Cell wall-associated ROOT HAIR SPECIFIC 10, a proline-rich receptor-like kinase, is a negative modulator of Arabidopsis root hair growth", JOURNAL OF EXPERIMENTAL BOTANY, vol. 67, no. 6, 16 February 2016 (2016-02-16), pages 2007 - 2022, XP055447937

## Description

### TECHNICAL FIELD

The present invention relates to a method for improving salt tolerance of a plant.

### BACKGROUND ART

In recent years, a need for a large amount of agricultural water has arisen due to the increase in food production accompanying the population increase in various countries around the world, and water shortage has become a serious problem. The water resource which is the most abundant on the earth is sea water, and if seawater can be used as agricultural water, this problem can be solved. However, most plants cannot be grown under the high salt concentration condition due to water absorption inhibition by osmotic pressure and inhibition of intracellular enzymes by sodium ions. If plants with low salt tolerance can be improved to have enhanced tolerance against salt up to concentration of the seawater level, such improved plants are expected to enable cultivation thereof to be done using seawater.

One example of method for enhancing the salt tolerance of plants is a method of introducing genes related to a salt tolerance mechanism by plant transformation techniques. For example, there are halophilous plants that have acquired resistance to osmotic pressure by accumulating osmolytes (proline or betaine) in their plant cells. It has been reported that a genetically-modified plant into which a gene to induce osmolyte accumulation has been introduced acquires salt tolerance.

Further, the intracellular sodium ion concentrations in plants are mainly regulated by non-selective cation channels (NSCC) that regulate uptake of cations into cells, a SOS pathway including a plasma membrane type Na⁺/H⁺ antiporter (Salt Overly Sensitive 1; SOS1) that regulates extracellular efflux of sodium ions, a vacuolar type Na⁺/H⁺ antiporter that regulates uptake of sodium ions into vacuoles, a high-affinity potassium transporter (High affinity K Transporter; HKT) that allows sodium ions to flow into cells with potassium ions via conduits (Non-Patent Document 1). For example, Patent Document 1 reports that a transformed plant overexpressing the SOS1 gene identified from a salt tolerant plant, Thellungiella halophila, exhibits promoted extracellular efflux of sodium ions and improved salt tolerance. Patent Document 2 reports that salt tolerance has been improved even in a transformed plant overexpressing the SOS2 gene, which is a protein kinase forming the SOS pathway. Patent Document 3 reports that a transformed plant overexpressing the gene encoding a vacuolar type Na⁺/H⁺ antiporter (HvNHX 1) of barley (Hordeum vulgare) exhibits promoted uptake of sodium ions into vacuoles and improved salt tolerance. Non-Patent Document 2 reports that a transformed plant overexpressing the HKT gene of Arabidopsis thaliana exhibits increased accumulation of sodium ions in the root, suppression of increase in the salt concentration of the shoot, and improved salt tolerance.

As regards a method for enhancing the salt tolerance of plants without gene manipulation, studies have been made on a method in which plants are administered with drugs or microorganisms which have an effect of imparting salt tolerance to plants. As a drug effective for imparting salt tolerance, for example, pyrroloquinoline quinone (see, for example, Patent Document 4) and strigolactones which are plant hormones and the like are known. Further, as a microorganism effective for imparting salt tolerance, for example, Paenibacillus fukuinensis is known (see, for example, Patent Document 5).

### Prior Art References

### Patent Document

Patent Document 1: International Patent Application Publication No. 2006/053246
Patent Document 2: International Patent Application Publication No. 2006/079045
Patent Document 3: Australian Patent Application Publication No. 2009201381
Patent Document 4: Japanese Patent Granted Publication No. 5013326
Patent Document 5: Japanese Unexamined Patent Application Publication No. 2013-75881

### Non-Patent Document

Non-Patent Document 1: Takeda and Matsuoka, Nature Reviews Genetics, 2008. Vol.9, p.444-457.
Non-Patent Document 2: Moller, et. al., The Plant Cell, 2009,Vol.21,p.2163-2178.
Non-Patent Document 3: Bai, et. al., The Plant Journal,2009,Vol.60,p.314-327.

The following non-patent documents relate to PERK mutants and transgenic plants:
Youra Hwang et al., "Cell wall-associated ROOT HAIR SPECIFIC 10, a proline-rich receptor-like kinase, is a negative modulator of Arabidopsis root hair growth", JOURNAL OF EXPERIMENTAL BOTANY, GB, (20160216), vol. 67, no. 6, pages 2007 - 2022.
Yosr Z Haffani et al., "Altered expression of PERK receptor kinases in Arabidopsis leads to changes in growth and floral organ formation", PLANT SIGNALING AND BEHAVIOUR, (20060901), vol. 1, no. 5, pages 251 - 260,
S.-K. Won et al., "cis-Element- and Transcriptome-Based Screening of Root Hair-Specific Genes and Their Functional Characterization in Arabidopsis", PLANT PHYSIOLOGY, Rockville, Md, USA, (20090515), vol. 150, no. 3, pages 1459 - 1473.
Ling Bai et al., "Plasma membrane-associated proline-rich extensin-like receptor kinase 4, a novel regulator of Ca 2+ signalling, is required for abscisic acid responses in Arabidopsis thaliana", THE PLANT JOURNAL, GB, (20091001), vol. 60, no. 2, pages 314 - 327.

### DISCLOSURE OF INVENTION

### Problems to be Solved by the Invention

As disclosed in Non-Patent Document 1, the mechanism underlying regulation of sodium ion concentration in plants has been elucidated to some extent. In addition, as described in Patent Document 1 and the like, it is known that salt tolerance of plants can be improved by overexpressing genes such as SOS1 gene, which are involved in the mechanism. However, most of the genetically modified plants to which genes involved in this mechanism have been introduced are confirmed to have tolerance against up to about 100 mM of sodium chloride, and further improvement of salt tolerance is desired.

It is an object of the present invention to provide a method for improving salt tolerance of a plant so as to enable the plant to be cultivated under a high salt concentration environment.

### Means to Solve the Problems

As a result of intensive research, the present inventors have found that PERK13 (Proline-rich extensin-like receptor kinase 13), the function of which wat not identified, is involved in the mechanism underlying regulation of sodium ion concentration in plants, and that inhibiting the function of PERK13 improves the salt tolerance of a plant. Based on this finding, the present invention has been completed.

The present invention provides the use of PERK13 in a plant for improving salt tolerance of the plant by suppressing or inhibiting a function of PERK13 in the plant as described in claim 1 (referred to in the following as "method of the present invention"), wherein the function of PERK13 is suppressed or inhibited such that, when the plant is cultivated hydroponically under the condition of a sodium chloride concentration of 1.0% by mass for 6 to 24 hours, an amount of sodium chloride in its root is 90% or less, relative to an amount (100%) of sodium chloride in the root when cultivating the plant under the same condition before suppressing or inhibiting the function of the PERK13.

Preferred embodiments of the present invention are subject-matter of dependent claims 2-12.

### Effect of the Invention

The method of the present invention for improving salt tolerance of a plant makes it possible to improve the salt tolerance of a plant which inherently has a low salt tolerance. Therefore, the resulting plant with its salt tolerance improved by this method can be cultivated even in an environment with a relatively high sodium chloride concentration.

### BRIEF DESCRIPTION OF THE DRAWINGS

FIG. 1 is a fluorescent stained image obtained with a fluorescent sodium indicator, showing the root of a wild-type of Arabidopsis thaliana which has been exposed to salt stress in Example 3.
FIG. 2 is a fluorescent stained image obtained with a fluorescent sodium indicator, showing a PERK13 function-deficient mutant of Arabidopsis thaliana which has been exposed to salt stress in Example 3.
FIG. 3 is a diagram showing the results of measurement of the fluorescence intensities with respect to the roots of the wild-type of Arabidopsis thaliana and the PERK13 function-deficient mutant of Arabidopsis thaliana, which have been exposed to salt stress in Example 3.
FIG. 4 is a diagram showing the results of measurement of survival rates of a wild-type of Arabidopsis thaliana and a PERK13 function-deficient mutant of Arabidopsis thaliana when hydroponically cultivated in Example 4 in symbiosis with an identified microbial mixture for improving the salt tolerance at various sodium chloride concentrations.
FIG. 5 is a diagram showing the results of measurement of the fluorescence intensities with respect to a wild-type of Arabidopsis thaliana and a PERK13 function-deficient mutant of Arabidopsis thaliana, which have been exposed to salt stress (sodium concentration of 2.5 % by mass) in Example 5 in the presence of the identified microbial mixture for improving the salt tolerance obtained in Example 4.
FIG. 6 is a diagram showing the results of measurement of the fluorescence intensities with respect to a wild-type of Arabidopsis thaliana and a PERK13 function-deficient mutant of Arabidopsis thaliana, which have been exposed to salt stress (sodium concentration of 1.0 % by mass) in Example 5 in the presence of the identified microbial mixture improving the salt tolerance obtained in Example 4.
FIG. 7 is a vector map of RNAi (pBI-SIPERKs-sense/antisense vector) targeting the tomato SlPERK gene, which was constructed in Example 6.
FIG. 8 is a photograph of a transgenic tomato obtained in Example 6 after 21 days from starting hydroponic cultivation under an environment of sodium chloride concentration of 0.5 or 1.0 % by mass.
FIG. 9 is a diagram showing the results of electrophoresis of a TspRI-treated a PCR product by using a genomic DNA as a template extracted from a recombinant regenerated plant in Example 7.
FIG. 10 is a photograph showing a recombinant regenerated plant (KO) into which a non-recombinant regenerated rice plant body (WT) and a vector for knockout of PERK13 ortholog gene had been introduced. The photograph was taken after cultivation of the recombinant regenerated plant (KO) in Example 7 under environment of a sodium chloride concentration of 1.5 % by mass for 2 weeks.

### DESCRIPTION OF THE EMBODIMENTS

The method of the present invention for improving salt tolerance of a plant includes suppressing or inhibiting function of PERK13 in a plant. As described in the Examples described later, in a mutant in which a mutation is introduced into the PERK13 gene to inhibit the function thereof, the influx of sodium ions into the plant from the root is suppressed, whereby the salt tolerance of the plant improves. PERK13 is a membrane protein specifically expressed in the root of a plant and has a kinase active site in the cell. From the similarity of the amino acid sequence, the PERK13 was presumed to have the same action as the PERK4 (Proline-rich extensin-like receptor kinase 4) which is a receptor that promotes the influx of calcium ions of NSCC (see, for example, Non-Patent Document 3). However, the researches made by the present inventors have revealed that the PERK13 is involved in regulation of the influx of sodium ions into the plant.

In the present specification, the phrase "expression of PERK13" or any similar phrase means a synthesis of PERK13 by expression of PERK13 gene.

As described in Non-Patent Document 1, the same mechanism for the sodium ion concentration regulation is shared by a wide variety of plants. PERK13 gene, which plays a part in this mechanism, is also a gene preserved in a wide variety of plants and the expression product thereof (PERK13) has a function to regulate the influx of sodium ions into plants in many plants. Examples of the PERK13 gene include an ortholog gene of the PERK13 gene of Arabidopsis thaliana with NCBI Gene ID: At1g70460 (Arabidopsis thaliana), and more specific examples include those with the following NCBI Gene IDs: 101266034 (Solanum lycopersicum), 107059185 (Solanum tuberosum), 107279382 (Oryza sativa Japonica Group), 4333279 (Oryza sativa Japonica Group), 102703815 (Oryza brachyantha), 103649394 (Zea mays), 106804357 (Setaria italica), 101775206 (Setaria italica), 106322706 Brassica oleracea), 106405068 (Brassica napus), 106416704 (Brassica napus), 103852653 (Brassica rapa), 101215732 (Cucumis sativus), 100247217 (Vitis vinifera), 104882493 (Vitis vinifera), 104822150 (Tarenaya hassleriana), 102616604 (Citrus sinensis), 105766022 (Gossypium raimondii), 104438961 (Eucalyptus grandis), 100807815 (Glycine max), 106779893 (Vigna radiata), 101497672 (Cicer arietinum), 103321809 (Prunus mume), 103927247 (Pyrus x bretschneideri), 104586282 (Nelumbo nucifera), 100832398 (Brachypodium distachyon), 103708226 (Phoenix dactylifera), 105049377 (Elaeis guineensis), 103989453 (Musa acuminata), 105172671 (Sesamum indicum), 104904972 (Beta vulgaris subsp. vulgaris), 18429568 (Amborella trichopoda), and 103452847 (Malus domestica).

In the method of the present invention for improving salt tolerance of a plant, there is no particular limitation on the PERK13 with its function to be suppressed or inhibited by the method as long as it has the function of the PERK13. In the method for improving salt tolerance of a plant according to the present invention, the PERK13 with its function to be suppressed or inhibited may be any of a wild-type PERK13 present in a wild-type plant, a PERK13 mutant resulted from mutation, a PERK13 mutant in which a mutation has been introduced by various mutagenesis treatment such as ultraviolet irradiation treatment, or a modified PERK13 modified by a gene modification technique. For example, even in the case of a plant possessing a modified PERK13 with its sodium ion influx promoting function enhanced or attenuated by various modifications of a wild-type PERK13, the method of the present invention can improve the salt tolerance of the plant.

The degree of improving salt tolerance by suppression or inhibition of the function of PERK13 in the plant thereof is as follows. When the plant is cultivated hydroponically under the condition of a sodium chloride concentration of 1.0% by mass for 6 to 24 hours, the PERK13 function is suppressed or inhibited such that the amount of sodium chloride in the root is 90% or less, preferably 80% or less, more preferably 60% or less, further preferably 50% or less, still more preferably 40% or less, especially preferably 30% or less, relative to the amount (100%) of sodium chloride in the root when cultivating the plant under the same condition before suppressing or inhibiting the function of the PERK13. The relative amount of sodium chloride in the root of the plant can be measured in terms of, for example, the fluorescence intensity obtained when the inside of the root is fluorescently stained with a fluorescent substance that binds to sodium ions.

There is no particular limitation on the method of improving salt tolerance in the plants possess PERK13 function. The suppression or inhibition may be effected by any of the following methods: a method of reducing the expression level of the PERK13, a method of introducing mutation into the PERK13 gene in the genomic DNA in order to decrease the PERK13 function, and a method of inhibiting intracellular signal transduction of the PERK13.

When the expression level of the PERK13 possessed by the plant is reduced in order to improve the salt tolerance of the plant, the reduced expression amount of the PERK13 in the plant is 90% or less, preferably 80% or less, more preferably 60% or less, still more preferably 50% or less, still more preferably 30% or less, especially preferably 0% (completely no expression), relative to the expression amount (100%) of the PERK13 in the plant before reducing the expression level. The expression level of the PERK13 in the plant can be measured by various methods used for measuring the level of gene expression for protein synthesis in the relevant techniques, such as the RT-PCR method. When the function of the PERK13 possessed by the plant is decreased in order to improve the salt tolerance of the plant, the decreased function of the PERK13 of the plant is 90% or less, preferably 80% or less, more preferably 60% or less, still more preferably 50% or less, still more preferably 30% or less, especially preferably 0% (completely no function), relative to the function (100%) of the PERK13 of the plant before decreasing the function.

The method of reducing the expression level of the PERK13 may be a method of modifying genomic DNA or a method without modification of the genomic DNA, such as RNA interference. Examples of the method for modifying the genomic DNA to reduce the expression level of the PERK13 include a method of deleting the PERK13 gene, a method of introducing a nonsense mutation into the PERK13 gene, a method of modifying an expression regulatory sequence such as a promoter sequence of the PERK13 gene. Examples of the mutations which decrease or delete the function of the PERK13 include a mutation in the kinase domain in the intracellular domain of the PERK13, which causes the kinase activity to disappear or decrease, and a mutation in the ligand binding site of the extracellular domain of PERK13, which decreases affinity to the ligand.

As a method of modifying the PERK13 gene region in the genomic DNA, a method of replacing all or a part of the PERK13 gene region with another DNA fragment by homologous recombination is widely used. For example, a mutant PERK13 can be caused to be expressed instead of a wild type PERK13 by replacing a region coding the PERK13 gene with a DNA fragment coding another gene or a DNA fragment having a mutated gene coding for the PERK13 into which a mutation has been introduced, to thereby delete the PERK13 gene or to cause a mutant PERK13 to be expressed instead of a wild-type PERK13. The homology (sequence coincidence) of the nucleotide sequence required for homologous recombination is preferably 70% or more, more preferably 80% or more, further preferably 90% or more, especially preferably 95% or more. The gene manipulation by homologous recombination method has already been established in many plants. For example, a genomic DNA with PERK13 gene region can be modified by introducing a transformation vector containing a DNA fragment for replacement by homologous recombination into a callus of a target plant for salt tolerance improvement, and allowing the resulting transformant to differentiate. Undifferentiated callus can be prepared by a conventional method. As a vector for transformation, a linear DNA or a plasmid may be used. The introduction of a vector into a plant cell such as callus can be carried out by any of various conventionally known methods such as Agrobacterium method, particle gun method, polyethylene glycol method, electroporation method, liposome method, calcium phosphate precipitation method, lipofection method, and microinjection method.

RNA interference can be carried out by introducing a siRNA (small interfering RNA), a shRNA (short hairpin RNA) or a miRNA (micro RNA) into the plant, wherein the siRNA has a double-stranded structure composed of a sense strand and an antisense strand of a partial region (target region for RNAi (RNA interference)) of cDNA of the PERK13 gene. An RNAi-inducing vector which is able to produce siRNA or the like may be introduced in a target plant cell. The siRNA, shRNA, miRNA, and RNAi-inducing vector can be designed and prepared by a conventional method from the base sequence information of cDNA of the target PERK13 gene. Further, the RNAi-inducing vector can also be prepared by inserting the nucleotide sequence of the RNAi target region into the nucleotide sequence of various commercially available RNAi vectors. Introduction of the RNAi-inducing vector can be carried out in the same manner as in the introduction of the above-mentioned transformation vector.

The inhibition of the intracellular signal transduction of PERK13 can be performed by a method of bringing an antagonist of PERK13 into contact with the surface of the root of a plant, or a method of introducing an inhibitor that inhibits the kinase activity of PERK13 into the root cell of a plant. The antagonist of PERK13 means a substance that binds to the extracellular domain of PERK13 and thereby inhibits the ligand of PERK13 from binding to PERK13. From the viewpoint of unnecessity of modification of the genomic DNA and higher simplicity of the treatment, an especially preferred method for improving the salt tolerance of a plant according to the present invention is a method of bringing the antagonist of PERK13 into contact with the surface of the root of the plant.

The antagonist of PERK13 used in the present invention may be any of a chemical compound, one or more microorganisms, and a secretion from one or more microorganisms. When the antagonist is a secretion from a specific microorganism, the culture supernatant of the microorganism as it is or a crudely purified product thereof may be brought into contact with the surface of the root of the plant.

As regards a method of bringing the antagonist of PERK13 into contact with the surface of the root of the plant, when cultivating the plant by hydroponic cultivation which is performed with at least a part of the root of the plant being immersed in a cultivation solution, the replacement of such a cultivation solution with a treatment solution containing an antagonist of PERK13 to immerse at least a part of the root of the plant in the treatment solution for a certain period allows the antagonist to be bound to the PERK13 on the surface of the root of the plant to inhibit the function of PERK13, thereby suppressing the influx of sodium ions from the root and improving the salt tolerance of the plant. The composition of the treatment solution, especially the composition of the salt, may be the same as or different from the cultivation solution. The antagonist of PERK13 may be directly mixed with the cultivating solution. When the plant is cultivated in the soil, the soil in which the plant is planted may be moistened with an aqueous solution containing the antagonist, or granules containing the antagonist may be placed near the roots in the soil.

In the present invention, the plant with its salt tolerance to be improved is not particularly limited as long as it is a plant inherently having the PERK13 gene or a homologue gene thereof in the genomic DNA. The plant may be either an angiosperms or a gymnosperm, and may even be a fern or a moss. Further, the plant may be a monocotyledonous plant or a dicotyledonous plant. Specific examples of the plant include plants of the Poaceae family such as rice, maize, sorghum, wheat, barley, rye, Japanese millet, and foxtail millet; plants of the Solanaceae family such as tomato, eggplant, paprika, green pepper, potato, and tobacco; plants of the Brassicaceae family such as Arabidopsis thaliana, rapeseed, shepherd's purse, Japanese white radish, cabbage, red cabbage, Brussels sprout (Petit vert), Chinese cabbage, bok-choy, kale, watercress, Japanese mustard spinach, broccoli, cauliflower, turnip, horseradish, and mustard; plants of the Cucurbitaceae family such as cucumber, bitter gourd, pumpkin, melon, and watermelon; plants of the Vitaceae family such as grapes; plants of the Rutaceae family such as lemons, oranges, navel oranges, grapefruit, mandarin, lime, sudachi, yuzu, Shiikuwasha, and Tankan; plants of the Rosacea family such as apple, cherry, Japanese apricot, peach, loquat, apricot, plum, prunes, almonds, Japanese pear, pear, strawberry, raspberry, blackberry, black currant, cranberry, and blueberry; plants of the Leguminosae family such as soy, kidney beans, peas, fava beans, green soy beans, mung bean, and chickpea; plants of the Nelumbonaceae family such as lotus (lotus root); plants of the Pedaliaceae family such as sesame; plants of the Chenopodiaceae family such as spinach, beet, sugar beet, quinoa, hiyu, amaranthus, and cockscomb; plants of the Palmae family such as date palm, oil palm, coconut, and acai; plants of the Musaceae family such as banana, Japanese banana, and Manila hemp; plants of the Malvaceae family such as cotton, and okra; plants of the Myrtaceae family such as eucalyptus; plants of the Capparidaceae family such as Cleome gynandra, and Cleome spinosa.

In the method of the present invention for improving the salt tolerance of the plant, in addition to suppression or inhibition of the function of PERK13, other treatments for lowering the sodium ion concentration in the cells of the root of the plant may be adopted as well. Examples of other treatments for lowering the sodium ion concentration in the cells of the root of the plant include a treatment for enhancing the plant with respect to a function of at least one protein selected from the group consisting of a nonselective cation channel, a plasma membrane Na⁺/H⁺ antiporter, a vacuolar Na⁺/H⁺ antiporter, and a high affinity potassium transporter. Examples of these proteins include SOS1, SOS2, SOS3, NHX1, and HKT1 (Non-Patent Document 1). The function of these proteins can be enhanced by increasing the expression level of the protein.

The expression level of the protein in the plant can be increased by introducing a foreign gene encoding the protein to thereby transform the plant. The foreign gene may be a gene derived from an organism of the same species as the plant to which the foreign gene is to be introduced or may be a gene derived from an organism of a different species. In the method of the present invention for improving salt tolerance of a plant, it is preferable to suppress or inhibit the function of PERK13 for a transformant into which at least one type of foreign gene has been introduced, wherein the foreign gene is derived from a plant of the same species as or different species from the plant with its salt tolerance to be improved, and is selected from the group consisting of SOS1 gene, SOS2 gene, SOS3 gene, NHX1 gene, and HKT1 gene. The introduction of a foreign gene into a plant can be carried out in the same manner as in the aforementioned introduction of a transformation vector, using a transformation vector to which a DNA fragment coding the foreign gene has been inserted.

The method of the present invention for improving salt tolerance of a plant makes it possible to obtain a plant having an improved salt tolerance as compared to a plant prior to suppression or inhibition of the function of PERK 13. In the method of the present invention for improving salt tolerance of a plant, it is preferable that the salt tolerance of a plant is improved to such an extent that the plant can grow even under environment of the sodium concentration at which only 10 to 50% of the plants prior to the improvement of salt tolerance can grow. It is more preferable that the salt tolerance of a plant is improved to such an extent that the plant can grow even under environment of the sodium concentration at which only 10 to 30% of the plants prior to the improvement of salt tolerance can grow. It is still more preferable that the salt tolerance of a plant is improved to such an extent that the plant can grow even under environment of the sodium concentration at which less than 10% of the plants prior to the improvement of salt tolerance can grow.

The plant obtained by the method of the present invention for improving salt tolerance of a plant can be cultivated by hydroponic cultivation using a cultivation solution having a high sodium ion concentration or soil cultivation using a soil having a high sodium ion concentration. For example, according to the method of the present invention for improving salt tolerance of a plant, it may be possible to obtain a plant which can be hydroponically cultivated even using a cultivating solution having a sodium ion concentration of 0.2 % by mass or more, preferably 0.5 % by mass or more, more preferably 1 % by mass or more, further preferably 1.5 % by mass or more, still more preferably 2.0 % by mass or more, especially preferably 2.5 % by mass or more.

The cultivation solution used for cultivation of a plant with improved salt tolerance preferably contains magnesium chloride in addition to sodium chloride. The cultivation solution more preferably contains 0.5% by mass or less of magnesium chloride, and still more preferably contains 1 to 0.5% by mass of magnesium chloride.

In addition to sodium chloride and magnesium chloride, the cultivation solution preferably contains various nutrients necessary for growing the plant. The nutrients can be appropriately adjusted according to the type of the plant to be cultivated. Especially, it is preferable that the cultivation solution contains elements necessary for the growth of the plant as salts. Examples of such elements include nitrogen, phosphorus, potassium, calcium, magnesium, sulfur, iron, manganese, copper, molybdenum, and boron. The cultivation solution may further contain elements such as aluminum and silicon as salts, depending on the type of the plant. Further, the composition of the cultivating solution may be changed according to the growing stage of the plant.

The cultivating solution to be used may be, for example, a solution prepared by supplementing deficient salt such as sodium chloride to commercially available liquid fertilizer or a solution obtained by diluting commercially available concentrated liquid fertilizer with sea water instead of fresh water. Further, the cultivation solution may also be a solution obtained by appropriately adding a deficient salt such as salt of phosphorus to seawater.

The hydroponic cultivation of a plant with improved salt tolerance can be carried out by a generally known hydroponic cultivation method. For example, the hydroponic cultivation may be carried out by a flooded type hydroponic method in which a relatively large amount of a cultivation solution is placed in a cultivation tank, or by a thin film hydroponic method in which a culture liquid is allowed to flow down little by little onto a flat surface having a gentle slope.

### Examples

Hereinbelow, the present invention will be described with reference to Examples which, however, should not be construed as limiting the present invention.

### [Example 1]

A library of mutants of Arabidopsis thaliana into which random mutations had been introduced was screened for mutants with improved salt tolerance, and genes contributing to the improvement of salt tolerance were searched. For this screening, microorganisms belonging to the genus Paenibacillus were used. Paenibacillus bacteria promote the influx of sodium chloride into the cells of Arabidopsis thaliana. For this reason, Arabidopsis thaliana coexisting with the Paenibacillus bacteria withers and dies even in an environment of 0.5 % by mass sodium chloride, which normally would not cause Arabidopsis thaliana to wither and die. Exploiting this characteristic, the screening was performed to identify genes that can improve salt tolerance even in the presence of Paenibacillus bacteria.

First, seeds of Arabidopsis thaliana (Col-0) were treated with EMS (ethyl methanesulfonic acid) to prepare a mutant library into which random mutations were introduced. The seeds of this library were sterilized with hypochlorous acid and then sown on a gel plate medium of MS (Murashige-Skoog) medium. Then, hydroponic cultivation was carried out with at least the bottom of the gel flat plate medium being in contact with a liquid medium for hydroponic cultivation. The liquid medium used was a 1/2 MS medium (liquid medium in which the MS medium was diluted with an equal amount of water).

When the germinated plants had become two weeks old, sodium chloride with a final concentration of 0.5% by mass was adjusted and Paenibasilus bacteria were added to a plant as a target for sodium chloride treatment, and hydroponic cultivation was further carried out for another 1 week. To a plant for control treatment was added only Paenibasilus bacteria, and hydroponic cultivation was further carried out for another 1 week.

Here, the expression "for control treatment" simply means that the plants were selected for sodium chloride-free cultivation experiments, and does not necessarily mean that the plants per se are outside the scope of the present invention.

As a result, the control wild-type withered and died due to the effect of coexistence with the Paenibacillus bacteria, but 10 plants out of about 25,000 random mutants survived and grew normally. Seeds were harvested from 4 plants of these 10 plants, and the seeds were grown into Arabidopsis thaliana plants. From leaves of the grown plants, the genome was extracted. The nucleotide sequence of the extracted genome was analyzed by a next generation sequencer. The results of the analysis revealed that the same mutation was introduced into the PERK13 genes (At1g70460) in all the 4 mutants. It was found that this mutation of the PERK13 gene was a single nucleotide insertion at the position of chr 5: 13434602, which caused a frame shift resulting in the loss of the PERK13 function in these mutants. Further, these 4 mutant strains shared no common mutation of genes other than in the PERK13 gene.

As shown in this experiment, in the mutants having a frame shift mutation in the PERK13 gene, the Paenibacillus bacteria's promotion effect on the sodium chloride influx into the plant was suppressed and the salt tolerance was improved. From these results, it was found that the suppression or inhibition of the function of PERK13 suppresses the influx of sodium ions from roots and improves the salt tolerance of a plant.

### [Example 2]

Two strains out of the four strains confirmed in Example 1 to be mutants deficient in the PERK13 function were cultivated under an environment of 1.5 % by mass sodium chloride, and the salt tolerance was evaluated.

Specifically, the seeds were sterilized with hypochlorous acid and then sown on a gel plate medium of MS medium. Then, hydroponic cultivation was carried out with at least the bottom of the gel flat plate medium being in contact with a liquid medium for hydroponic cultivation. The liquid medium used was a 1/2 MS medium. With respect to each of the mutants, 24 seeds for each sodium chloride treatment and 24 seeds for control treatment were sowed. When the germinated plants had become two weeks old, sodium chloride with a final concentration of 1.5% by mass was added to a plant as a target for sodium chloride treatment, and hydroponic cultivation was further carried out for another 1 week. A plant for control treatment was further hydroponically cultivated for another 1 week without adding anything. As a control, wild-type plants were likewise hydroponically cultivated.

As a result, in the case of the control treatment, i.e., cultivation in the 1/2 MS medium without addition of sodium chloride, all of the 24 plants of each of the wild-type plants and the PERK13 function-deficient mutants had survived and grown normally. With respect to the plants subjected to the sodium chloride treatment, 20 out of the 24 plants of the wild-type withered and died ([number of dead plants] / [total number of plants] = 20/24), whereas, in each of the two strains of the PERK13 function-deficient mutants, the number of dead plants was 4 or less, while most of the plants survived and grew in the liquid medium containing 1.5 % by mass sodium chloride ([number of dead plants] / [total number of plants] ≤ 4/24). From these results, it was confirmed that the suppression or inhibition of the function of PERK13 suppresses the influx of sodium ions from roots and improves the salt tolerance of a plant.

### [Example 3]

With respect to the PERK13 function-deficient mutants, the amount of sodium in roots was examined for one of the two strains examined for salt tolerance in Example 2.

Specifically, the seeds were sterilized with 70% ethanol and hypochlorous acid and then sown on a gel plate medium of 1% sucrose-containing MS medium. Then, hydroponic cultivation was carried out in a growth chamber with at least the bottom of the gel flat plate medium being in contact with a liquid medium (1/2 MS medium) . The operation conditions of the growth chamber were as follows: temperature of 25 °C, illuminance of 5000 lux, light period of 16 hours, and dark period of 8 hours. 10 to 14 days after germination, the liquid medium in contact with the bottom of the gel plate medium was changed to a 1/2 MS medium containing sodium chloride at the final concentration of 1.0% by mass, and hydroponic cultivation was further carried out for another 6 to 24 hours to apply salt stress to the plants. The plants for control treatment were cultivated for the same period without adding anything to the liquid medium.

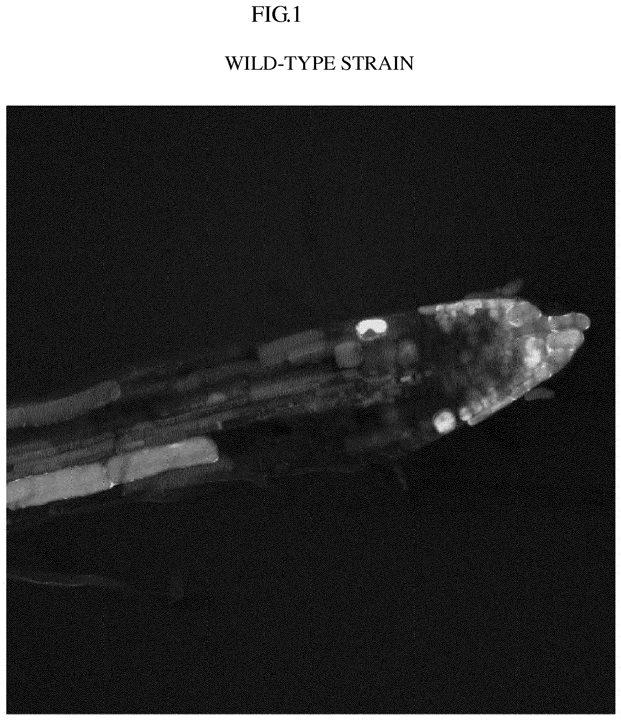

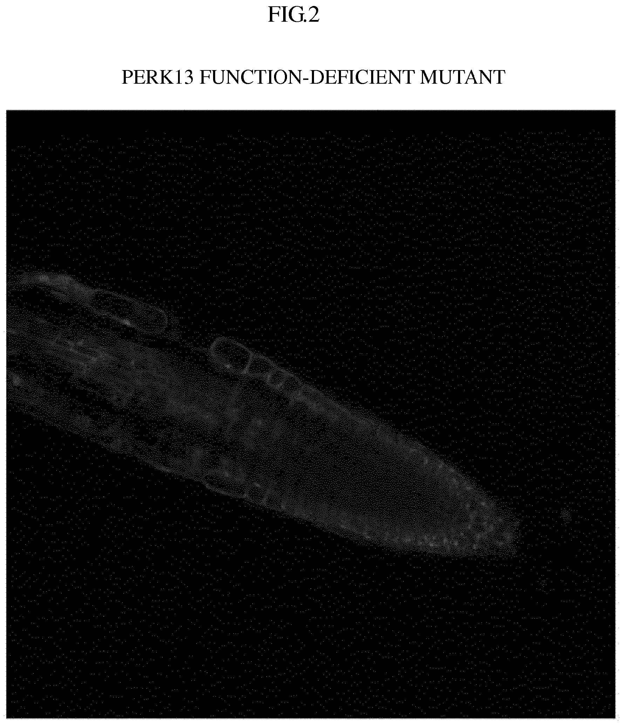

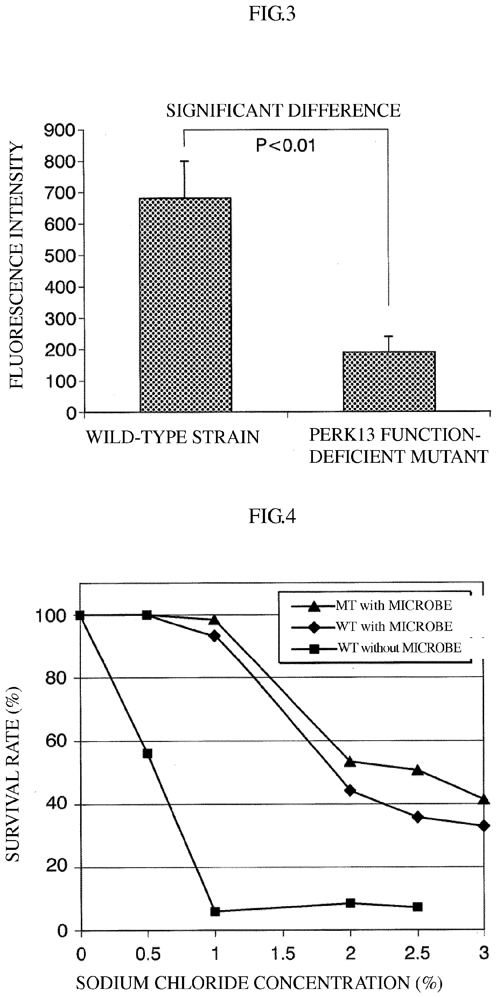

A root of the plant after application of salt stress was fluorescently stained with 50 µM of CoroNa (registered trademark) -Green AM solution, and the surface of the root was washed with water. The interior of the root after washing was observed with a confocal laser microscope. CoroNa-Green AM is a sodium indicator that increases green fluorescence intensity by binding with sodium ions. With respect to the wild-type strain and the PERK13 function-deficient mutant, the fluorescence stained images of the roots after application of salt stress are shown in FIG. 1 and FIG. 2. Further, with respect to the wild-type strain and the PERK13 function-deficient mutant, the measurement results of the fluorescence intensity per unit cross-sectional area of the root after application of salt stress are shown in FIG. 3.

In the roots of the plants for control treatment that had not been subjected to salt stress, almost no green fluorescence was observed for both of the wild-type strain and the PERK13 function-deficient mutant (not shown). In contrast, in the roots of the wild-type strain (FIG. 1), strong green fluorescence was observed, whereby it was confirmed that the influx of sodium ions remarkably increased due to salt stress. On the other hand, in the roots of PERK13 function-deficient mutant (Fig. 2), almost no green fluorescence was observed, whereby it was confirmed that the influx of sodium ions did not increase even by salt stress. From these results, it was found that the PERK13 is involved in the influx of sodium ions in the roots, and the improvement in tolerance against salt stress in the PERK13 function-deficient mutant is attributable to suppression of influx of sodium ions into the plant under high salt concentration.

### [Example 4]

Using a wild-type Arabidopsis thaliana, a group of plant symbiotic bacteria having a symbiotic effect to increase salt tolerance was selected from microorganisms extracted from the soil.

### <Preparation of Microbial Suspension>

1 g of soil collected in Okinawa prefecture was suspended in a buffer solution, and the resulting was thoroughly stirred to obtain a microbial suspension.

### <Preparation of Pots>

A sucrose-containing MS agar medium (medium prepared by adding 0.5% (w/v) sucrose and 0.9% (w/v) agar to a MS medium) was injected into a cylindrical pot with open top and bottom, and the agar medium was solidified to prepare a pot for growing a plant. A plurality of thus prepared pots were placed in each of eight containers containing a sucrose-containing MS medium (liquid medium prepared by adding 0.5% (w/v) sucrose to a MS medium).

### <Hypochlorous Acid Treatment of Seeds>

Arabidopsis thaliana seeds (Col-0) were purchased from LEHLE (Round Rock, TX, USA). The seeds were stirred for 1 minute while being immersed in 1% hypochlorous acid solution to thereby sterilize the surfaces of the seeds, followed by removal of hypochlorous acid by centrifugation. After the hypochlorous acid treatment, the seeds were washed three times with sterilized water, sown on the top of the pot, and stored in a dark place at 4 °C for 24 hours.

### <Hydroponic Cultivation of Plants>

A plurality of the above pots were prepared and all of them were placed in one container containing a sucrose-containing MS medium (liquid medium prepared by adding 0.5% (w/v) sucrose to a MS medium). Each pot was installed such that the bottom surface thereof was immersed in the sucrose-containing MS medium while the top surface thereof was not immersed. On the top of these pots, seeds of wild-type after washing three times with sterilized water after hypochlorous acid treatment were sown and grown at 25 °C for 14 days in an incubator under long day conditions with 16 hours of light and 8 hours of darkness.

### <Salt Stress and Inoculation of Microorganisms>

14 days after initiating the hydroponic cultivation, a sterilized 5 M sodium chloride aqueous solution was added to the sucrose-containing MS medium in which the bottom of the pot was immersed, such that the final concentration of sodium chloride became 1 % by mass, followed by addition of 100 µl of the microbial suspension. Thereafter, the pot was cultured for 14 days.

### <Recovering Microorganism Having Effect of Improving Tolerance Against Salt Stress>

After cultivation under salt stress for 14 days, the roots and the above-ground parts (leaves and stems) of the growing plants were cut, and the roots were collected and homogenized to obtain a first microorganism recovery solution.

The hydroponic cultivation was carried out in the same manner as mentioned above except that the cultivation solution used was a solution prepared by adding 100 µL of the first microorganism recovery solution to a sucrose-containing MS medium to which sodium chloride had been added such that the final concentration of sodium chloride became 1.5% by mass. After 14 days of cultivation under salt stress, the roots and the above-ground parts (leaves and stems) of the growing plants were cut, and the roots were collected and homogenized to obtain a second microorganism recovery solution.

The hydroponic cultivation was carried out in the same manner as mentioned above except that the cultivation solution used was a solution prepared by adding 100 µL of the second microorganism recovery solution to a sucrose-containing MS medium to which sodium chloride had been added such that the final concentration of sodium chloride became 3.0% by mass. After 14 days of cultivation under salt stress, the roots and the above-ground parts (leaves and stems) of the growing plants were cut, and the roots were collected and homogenized to obtain a third microorganism recovery solution.

The symbiosis in the roots of the plants with the microbial mixture contained in the third microorganism recovery solution enabled Arabidopsis thaliana to grow under salt stress. That is, it has been found that the microbial mixture contained in the third microorganism recovery solution or the secretion therefrom has an action of improving the salt tolerance of plants, that is, the microbial mixture is a group of plant symbiotic bacteria that enable a plant to grow under salt stress.

### <Identification of Microorganisms>

The microorganisms forming the screened group of plant symbiotic bacteria (microbial mixture for improving salt tolerance) that enable a plant to grow under salt stress were identified.

First, bacterial cells were recovered from the third microorganism recovery solution, and a genomic DNA was obtained from a part of the recovered cells using a GenElute Bacterial Genomic DNA kit (Sigma-Aldrich, St. Louis, MO, USA).

Using the recovered genomic DNA as a template, 16S rDNA was amplified by PCR using a forward primer (5'-AGAGTTTGATCATGGCTCAG-3 ', SEQ ID NO: 1) and a reverse primer (5'- TACGGTTACCTTGTTACGACTT-3', SEQ ID NO: 2). The temperature conditions of PCR were as follows: a heating step at 95 °C for 3 minutes; subsequent 30 cycles of a sequence of a denaturation step at 95 °C for 30 seconds, an annealing step at 50 °C for 30 seconds, and an elongation step at 72 °C for 1 minute and 30 seconds; and a final elongation reaction at 72 °C for 5 minutes. The obtained PCR product was confirmed by 1.2% agarose gel electrophoresis and extracted from the gel using a QIAquick gel extraction kit (Quiagen, Germantown, MD, USA). The extracted PCR product was inserted into a plasmid using TOPO-TA cloning kit (Life Technologies, Carlsbad, Calif., USA), and transformed into E. coli. Thirty (30) E. coli colonies cultured overnight on ampicillin-containing LB plate medium were randomly picked and transplanted into ampicillin-containing LB liquid medium, followed by culturing. A plasmid was purified from E. coli cultured using QIAprep spin miniprep kit (Quiagen). The purified plasmid was subjected to thermalcycle reaction using BigDye terminator v3.1 Cycle sequence kit (Life Techonologies), and the base sequence of 16S rDNA inserted in the plasmid was determined with a DNA sequencer (ABI 3130 x L) . As a result, two types of 16S rDNA (YROK-1 strain and YROK-2 strain) were identified.

EzBIO Cloud search was conducted for the base sequences of two types of 16S rDNAs, the sequences of which had been determined. As a result, YROK-1 strain (SEQ ID NO: 3) had a sequence homology of 98.89% with Paenarthrobacter nitrogua jacolicus (accession number: AJ 512504) , and YROK-2 strain (SEQ ID NO: 4) had a sequence homology of 97.14% with Arthrobacter psychrochitiniphilus (accession number: AJ810896). From these results, it was found that the YROK-1 strain is a novel strain of Paenarthrobacter nitroguajacolicus and the YROK-2 strain is a novel strain of Arthrobacter psychrochitiniphilus.

Further, when the abundance ratio of these two types of microorganisms was examined from the ratio of 16S rDNA inserted in the identified 52 transformants, the abundance ratio of the Paenarthrobacter nitroguajacolicus YROK-1 strain was found to be 98.0% and the abundance ratio of the Arthrobacter psychrochitiniphilus YROK-2 strain was found to be 2.0%.

### <Symbiotic Effect Enhancing Salt Tolerance of Identified Microbial Mixture for Improving Salt Tolerance>

With respect to the wild-type strain and the PERK13 function-deficient mutant of Arabidopsis thaliana, the salt tolerance improving effect of the identified microbial mixture for improving salt tolerance was evaluated.

First, plants which had been cultivated by pots for 14 days were prepared in the same manner as in the above <Hydroponic Cultivation of Plants>. To the sucrose-containing MS medium in which the bottom of the pot was immersed, a sterilized 5 M sodium chloride aqueous solution was added such that the final concentration of sodium chloride became 0, 0.5, 1.0, 1.5, 2.0, 2.5 or 3.0% by mass, followed by addition of the above microbial mixture for improving salt tolerance. Then, hydroponic cultivation was carried out, and the survival rate after 14 days of cultivation was examined. As a control, hydroponic cultivation was likewise carried out in a cultivation solution to which the microbial mixture for improving salt tolerance was not added, and the survival rate after 14 day of cultivation was examined.

The measurement results of the survival rate are shown in FIG. 4. In FIG. 4, "WT without MICROBE" shows the results as to the wild-type strain cultivated without symbiosis with the microbial mixture for improving salt tolerance, "WT with MICROBE" shows the results as to the wild-type strain cultivated in symbiosis with the microbial mixture for improving salt tolerance, and "MT with MICROBE" shows the results as to the PERK13 function-deficient mutant cultivated in symbiosis with the microbial mixture for improving salt tolerance.

As a result, the wild-type strain without symbiosis with the microbial mixture for improving salt tolerance exhibited a survival rate of 10% or less when the sodium chloride concentration was 1% by mass, whereas, as in the case of the PERK13 function-deficient mutant, the wild-type strain in symbiosis with the microbial mixture for improving salt tolerance exhibited a survival rate as high as 90% or more when the sodium chloride concentration was 1% by mass, and exhibited a survival rate as high as 30% or more even when the sodium chloride concentration was 3 % by mass. As shown in FIG. 4, the PERK13 function-deficient mutant exhibited slightly higher survival rate than the wild-type strain when cultivated in symbiosis with the microbial mixture for improving salt tolerance and with the sodium chloride concentration of 2% or more. As to the reason for this result, it is speculated that the microbial mixture for improving salt tolerance not only has an action to improve salt tolerance through the deletion of the PERK13 function but also has some action to improve salt tolerance through other pathway.

The survival curves of the wild-type strain and the PERK13 function-deficient mutant are almost identical when these were cultivated in symbiosis with the microbial mixture for improving salt tolerance; therefore, the salt tolerance improvement effect of the microbial mixture on the wild-type strain is considered to be an effect conferred by deletion of the PERK13 function. In other words, it was suggested that the microbial mixture for improving salt tolerance or its secretion is an antagonist of PERK13, and the symbiosis in the roots of the plants with the microbial mixture suppresses or inhibits the function of PERK13, and it was also suggested that a salt tolerance improvement effect similar to the case of the PERK13 function-deficient mutant can be obtained by contacting the antagonist of PERK13 with the surface of the roots of the plants.

### [Example 5]

With respect to the wild type strain and the PERK13 function-deficient mutant of Arabidopsis thaliana, the influence of the microbial mixture for improving salt tolerance obtained in Example 4 on the sodium influx into the roots of the plant under salt stress was examined.

Firstly, with respect to each of the wild type strain and the PERK13 function-deficient mutant of Arabidopsis thaliana, the seeds were sterilized with 70% ethanol and hypochlorous acid and then sown on a gel plate medium of 1% sucrose-containing MS medium. Then, hydroponic cultivation was carried out in an artificial climate chamber with at least the bottom of the gel flat plate medium being in contact with a liquid medium (1/2 MS medium) for hydroponic cultivation. The operation conditions of the climatic chamber were as follows: temperature of 25 °C, illuminance of 5000 lux, and a long day condition with light period of 16 hours and dark period of 8 hours. 10 to 14 days after germination of the seeds, the liquid medium in contact with the bottom of the gel plate medium on which the plants were placed was changed to a 1/2 MS medium containing sodium chloride at the final concentration of 2.5% by mass or 1.0% by mass, followed by addition of the microbial mixture for improving salt tolerance. Then, hydroponic cultivation was carried out for 6 hours to apply salt stress in the presence of the microbial mixture for improving salt tolerance.

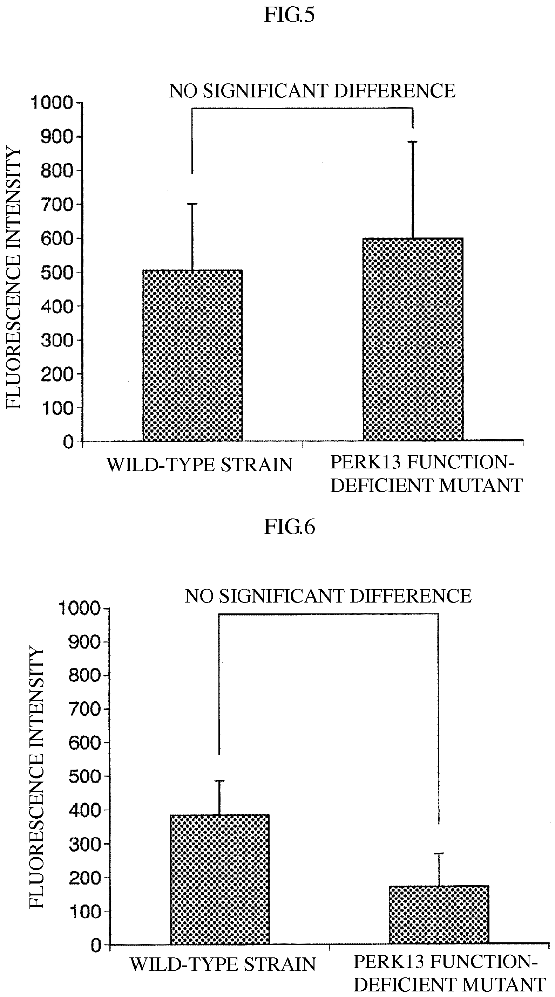

The root of the plant after application of salt stress was stained with a fluorescent sodium indicator (CoroNa-Green AM) in the same manner as in Example 3, and the fluorescence intensity per unit cross-sectional area of the root after application of salt stress was measured with respect to the wild-type strain and the PERK13 function-deficient mutant. FIG. 5 shows the results of hydroponic cultivation of the plants at a sodium chloride concentration of 2.5% by mass, and FIG. 6 shows the results of hydroponic cultivation of the plants at a sodium chloride concentration of 1.0% by mass.

As a result, in the case of cultivation in the presence of the microbial mixture for improving salt tolerance obtained in Example 4, there was no significant difference in the fluorescence intensity per unit cross-sectional area of the root between the wild-type strain and the PERK13 function-deficient mutant, meaning that the intensity values thereof were almost the same. From these results, it has been found that in the wild-type strain, the sodium influx into the roots under the salt stress is suppressed by the above microbial mixture for improving salt tolerance as in the case of the PERK13 function-deficient mutant, that this suppression of sodium influx into the roots improves the tolerance against salt stress of the wild type strain up to the same level as in the case of the PERK13 function-deficient mutant, and that the above microbial mixture for improving salt tolerance has an action to suppress or inhibit the function of PERK13.

### [Example 6]

A PERK13 function-deficient mutant of tomato (Solanum lycopersicum) was prepared, and the salt tolerance thereof was evaluated.

### <Selection of Target Gene>

Of the genes contained in the genomic DNA of tomato, three genes (PERK13 ortholog genes of tomato) having at least 60% sequence homology to PERK13 of Arabidopsis thaliana, i.e., SlPERK9b (Solyc05g010140.2.1), SlPERK10 (Solyc01g010030.2.1) and SlPERK9a (Solyc04g006930.2.1), were selected as target genes for constructing an RNAi vector. The genes correspond to amino acid sequences with identity of 98% or more and, hence, are thought to be paralogs of the PERK13 ortholog gene of tomato (Gene ID of NCBI: 101266034). The target sequence was 200 bases from the 5' end side of the gene translation region. Also, in order to make the genes simultaneously a target sequence of RNAi, a chimeric gene was prepared which had a base sequence formed by combining the target sequence (SEQ ID NO: 5) of the SlPERK10 gene, the target sequence (SEQ ID NO: 6) of the SlPERK9a gene, and the target sequence (SEQ ID NO: 7) of the SlPERK9b gene.

### <Vector Construction>

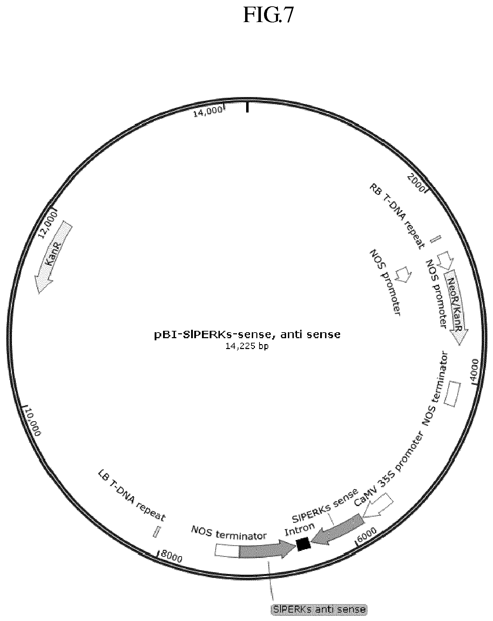

The artificially synthesized chimeric gene described above was introduced into a modified vector of pBI-sense, anti-sense-GW vector (manufactured by Clontech) by homologous recombination. Specifically, the chimeric gene was cloned between the cauliflower mosaic virus 35 S (CaMV 35 S) promoter and the expression cassette of the nopaline synthase gene terminator sequence (NOS) of the modified vector in the sense and antisense directions, respectively, to thereby construct an RNAi vector (pBI-SlPERKs-sense/antisense vector) targeting the SlPERK gene. The structure map of the vector is shown in FIG. 7. An RNA of the chimeric gene transcribed under the control of the CaMV 35S promoter formed a double-stranded RNA consisting of a sense RNA and an antisense RNA via cleavage of the intron.

### <Transformation of Tomatoes>

The prepared RNAi vector was introduced into Agrobacterium tumefaciens GV 3101 strain by a conventional method to obtain a recombinant Agrobacterium. Callus formation was induced in the callus formation medium by infecting a cotyledon piece derived from Micro-Tom (a tomato cultivar) with the obtained recombinant Agrobacterium. Thereafter, drug resistant calli were selected and redifferentiated.

DNA was extracted from the leaves of the tomatoes obtained by redifferentiation and subjected to PCR to select transgenic tomatoes into which the chimeric gene had been introduced. DNA extraction from the leaves and PCR were carried out as follows.

### <DNA Extraction>

100 mg of a plant sample was frozen in liquid nitrogen and powdered. A sample solution was prepared by adding 300 µL of extraction buffer (100 mM Tris, 50 mM EDTA, 500 mM NaCl (pH 8.0)) and 15 µL of 20% SDS to the obtained powder, followed by stirring. The resulting sample solution was incubated at 65 °C for 10 minutes. After the incubation, 90 µL of 5 M potassium acetate was added to the sample solution, followed by centrifugation at 14,000 rpm for 10 minutes. The supernatant was transferred to another tube, followed by addition of 400 µL of isopropanol. The resulting mixture was allowed to stand at room temperature for 2 minutes and then centrifuged at 14,000 rpm for 2 minutes. The resulting pellets were washed with 500 µL of 70% ethanol, dried and dissolved in 100 µL of water to prepare a DNA sample.

### <PCR>

PCR was performed using a GoTaq polymerase (manufactured by Promega), while adjusting a PCR reaction liquid such that the final concentration of each of a forward primer (5'-GTTCTTCTACACCATTTGCAGC, SEQ ID NO: 8) and a reverse primer (5'-ATTGTGGTAGTGTTGGTAAGGC, SEQ ID NO: 9) became 0.2µM. PCR was performed under the following thermal cycle conditions: heating at 95 °C for 3 minutes; subsequent 35 cycles of a sequence of heating at 95 °C for 30 seconds, heating at 55 °C for 30 seconds, and heating at 72 °C for 30 seconds which were performed in this order; and final heating at 72 °C for 3 minutes.

### <Evaluation of Salt Resistance>

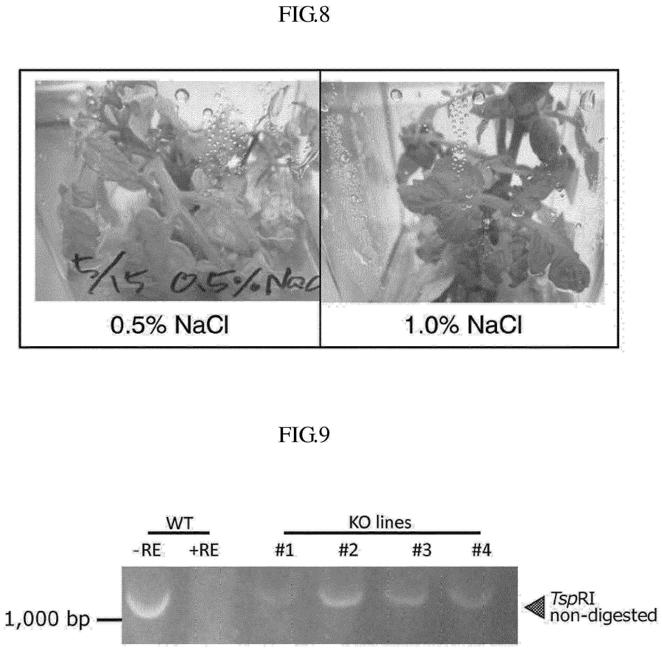

The resulting transgenic tomato was a tomato from which the function of tomato PERK13 (SlPERK) had been deleted by the introduced chimeric gene. This PERK13 function-deficient tomato was hydroponically cultivated in a 1/2 MS medium to which sodium chloride had been added such that the final concentration thereof became 0.5, 1.0, 1.5 or 2.0 % by mass. The hydroponic cultivation was carried out in an artificial climate chamber (25 °C, light period of 16 hours, and dark period of 8 hours). In the case of the wild-type tomato, the hydroponic cultivation in the 1/2 MS medium with a final sodium chloride concentration of 0.5% by mass resulted in whitened leaves, browned roots and withering of the plant on the day 21 of cultivation (not shown), as the plant could not tolerate the high concentration of sodium chloride. By contrast, in the case of the PERK13 function-deficient tomatoes cultivated in the 1/2 MS media containing 0.5 and 1.0% by mass of sodium chloride, there were plants confirmed to have grown without leaf whitening (Fig. 8). Further, the leaf whitening was observed in the PERK13 function-deficient tomatoes cultivated in the 1/2 MS media containing 1.5 and 2.0% by mass of sodium chloride, which however had not suffered from root browning. From these results, it has been found that the suppression or inhibition of the function of PERK13 can improve the salt tolerance of tomatoes as well.

### [Example 7]

A PERK13 function-deficient mutant of rice (Oryza sativa) was prepared, and the salt tolerance thereof was evaluated.

### <Selection of Target Gene and Construction of Vector for Knockout>

Of the genes contained in the genomic DNA of rice, a gene (PERK13 ortholog gene of rice) corresponding to an amino acid sequence of sequence identity of at least 70% relative to PERK13 of Arabidopsis thaliana, i.e., OsPERK13 (Os03g056880, NCBI GeneID: 4333279), was selected as a target to be knocked out. In order to make this gene a target sequence for knockout, a polynucleotide corresponding to a target sequence (SEQ ID NO: 10) of the OsPERK13 gene was prepared by artificial gene synthesis. The knockout vector pOsPERK-KO1 targeting the OsPERK13 gene was constructed by introducing the artificially synthesized polynucleotide into a modified pRIT1 vector (Terada et al., Nature Biotechnology, 2002, vol. 20, p. 1030-1034) by homologous recombination.

### <Transformation of Rice>

Transformation of rice was carried out according to the method of Toki et al. (Plant Journal, 2006, 47, 69-76). First, the knockout vector was introduced into Agrobacterium strain EHA 101 or LBA 4404 by a conventional method to obtain a recombinant Agrobacterium. The obtained recombinant Agrobacterium was infected with a scutellum-derived callus of rice cultivar "Nipponbare". The infected rice callus was cultured on a solidified medium containing 0.25 µM bispyribac-sodium salt, and calli were selected.

Genomic DNA was extracted from the selected bispyribac salt tolerant calli using a DNA extraction kit "Maxwell 16 LEV Plant DNA kit" (manufactured by Promega), and PCR was carried out to select transformed calli into which the knockout vector had been introduced. PCR was carried out using a DNA polymerase (Tks Gflex, Takara Bio), a forward primer (5'-AAGCTCAAGCTCCAATACGCAAACCGCCTC, SEQ ID NO: 11) and a reverse primer (5'-GACGGTATCGATAAGCTTGGCGCGCCATTA, SEQ ID NO: 12) under the following thermal cycle conditions: heating at 94 °C for 1 minute; subsequent 35 cycles of a sequence of heating at 98 °C for 10 seconds, heating at 60 °C for 15 seconds, and heating at 68 °C for 1 minute which were performed in this order; and final heating at 68 °C for 7 minutes. The bispyribac-sodium salt tolerant calli resulted in a PCR product of a desired size were selected as transformed calli into which the knockout vector had been introduced.

### <Regeneration of Rice Plants>

The rice calli that were confirmed to have the knockout vector introduced were subcultured to a regeneration medium and cultured in a bright place at 25 °C for about 3 weeks. As a result, transgenic regenerants carrying pOsPERK-KO1 were obtained. Likewise, non-transgenic regenerants were also acquired from non-transgenic rice calli.

### <Confirmation of Knockout Status of Rice PERK13 Ortholog Gene

The knockout status of OsPERK13 gene in the recombinant regenerated plants was analyzed by CAPS (Cleared Amplified Polymorphic Sequences) method. First, genomic DNA was extracted from each plant using a DNA extraction kit "Maxwell 16 LEV Plant DNA kit" (manufactured by Promega). The, PCR was performed using 3g05688 No1-F primer (5 '-AGTCAAGCTTCGCCGGCGCCAATGCCGATGTGAGCCCGGC, SEQ ID NO: 13) which is a 3g05688-specific primer, and 3g05688 No1-R primer (5' - TGACGAATTCGCTCCGGCACGACGAGGGTTCTCCTGCGCG, SEQ ID NO: 14). The obtained PCR amplification product was purified using a nucleic acid purification kit "DNA Cleaner" (manufactured by Wako Pure Chemical Industries, Ltd.), followed by restriction enzyme (TspRI) treatment, and the cleavage state of the DNA fragment was confirmed by agarose electrophoresis.

When a target gene is knocked out, a DNA sequence including a recognition sequence for a restriction enzyme present in PCR amplified fragments will be lost; therefore, the knockout status can be judged based on the presence of the uncleaved PCR amplification product. FIG. 9 shows the results of agarose gel electrophoresis of digested products obtained by treating OsPERK13 gene-derived PCR amplified fragments with TspRI. In FIG. 9, "-RE" of "WT" is a lane to which a PCR amplified fragment of a non-transgenic regenerated plant was applied, "+RE" of "WT" is a lane to which a digested product obtained by the TspRI treatment of a PCR amplified fragment of a non-transgenic regenerated was applied, and each of "#1" to "# 4" of "KO lines" is a lane to which a digested product obtained by the TspRI treatment of a PCR amplified fragment of recombinant regenerated plant into which the vector for knockout of OsPERK13 gene had been inserted was applied. As a result of the CAPS analysis , as shown in FIG. 9, the band observed with "-RE" of "WT" was not observed in "+RE" of "WT", whereas the band of undigested PCR amplified fragment was detected in "#1" to "# 4" of "KO lines" From this result, it was confirmed that the OsPERK13 gene was knocked out in plural transgenic plants .

### <Evaluation of Salt Tolerance of Rice PERK 13 Ortholog Gene Knockout Variant>

Each of a transgenic plant carrying pOsPERK-KO1 a non-transgenic plant was individually subcultured on a rooting medium (solid 1/2 MS) containing 1.5% by mass of sodium chloride. The plants were grown in a growth chamber (25 °C, constant light period) for about two weeks, and their phenotypes of the plants were analyzed.

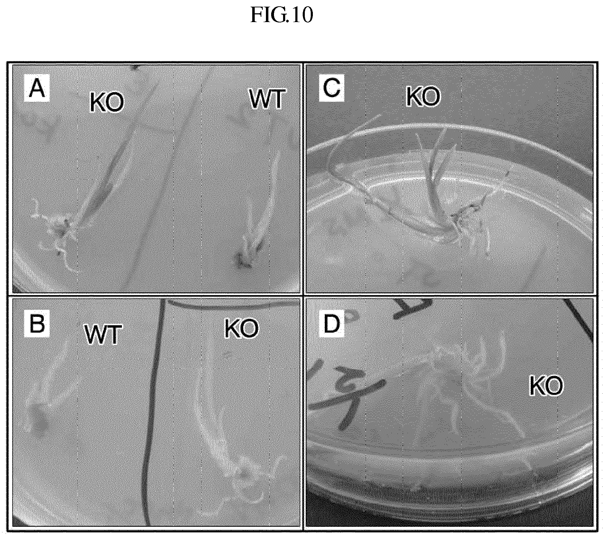

The phenotypes of 2-week-old regenerated rice plants are shown in FIG. 10. In FIG. 10, "WT" is a non-recombinant regenerated plant and "KO" is a recombinant regenerated plant into which the vector for knockout of PERK13 ortholog gene had been introduced. FIG. 10(A) and FIG. 10(C) are indicating the aerial part of plants, FIG. 10(B) and FIG. 10(D) indicate the underground part of the plants shown in FIG. 10(A) and FIG. 10(C), respectively. As shown in FIG. 10(A) and FIG. 10(B), non-transgenic plants showed typical phenotypes caused by sodium chloride stress with yellowed leaves, necrosis at basement and severe growth inhibition of roots. On the contrary, the transgenic plants harboring pOs PERK-KO1 showed normal growth with green leaves and root elongation even though some leaves turned to yellow. Furthermore, as shown in FIG. 10(C) and FIG. 10(D), one transgenic plant harboring pOsPERK-KO1 showed very healthy growth without any yellowing leaves. These results confirmed that plant salt tolerance improves with disruption of OsPERK13 gene, in which the OsPERK13 gene is a rice orthologue gene of PERK13, and such plants can be grown even under environment of 1.5% by mass sodium chloride, that is, the suppression or inhibition of the function of PERK13 can improve the salt tolerance of the rice plants as well.

## Claims

1. A use of PERK 13 (Proline-rich extensin-like receptor kinase 13) in a plant for improving salt tolerance of the plant by suppressing or inhibiting a function of PERK 13 in the plant, wherein the function of PERK13 is suppressed or inhibited such that, when the plant is cultivated hydroponically under the condition of a sodium chloride concentration of 1.0% by mass for 6 to 24 hours, an amount of sodium chloride in its root is 90% or less, relative to an amount (100%) of sodium chloride in the root when cultivating the plant under the same condition before suppressing or inhibiting the function of the PERK13.

2. The use according to claim 1, wherein the suppression or inhibition of the function of the PERK13 is carried out by suppressing or inhibiting expression of the PERK13 gene or PERK13 protein.

3. The use according to claim 1 or 2, wherein the suppression or inhibition of the function of the PERK13 is carried out by introducing a mutation into the plant to decrease or disrupt function of its PERK13 gene.

4. The use according to any one of claims 1 to 3, wherein the plant is enhanced with respect to a function of at least one protein selected from a nonselective cation channel, a plasma membrane Na⁺/H⁺ antiporter, a vacuolar Na⁺/H⁺ antiporter, and a high affinity potassium transporter.

5. The use according to any one of claims 1 to 3, wherein the plant has overexpression of a gene to produce at least one protein selected from a nonselective cation channel, a plasma membrane Na⁺/H⁺ antiporter, a vacuolar Na⁺/H⁺ antiporter, and a high affinity potassium transporter.

6. The use according to any one of claims 1 to 3, wherein the plant is a transformant into which a foreign gene has been introduced,
the foreign gene being at least one of genes selected from SOS1 gene, SOS2 gene, SOS3 gene, NHX1 gene, and HKT1 gene.

7. The use according to any one of claims 1 to 6, wherein the plant is a dicotyledonous plant.

8. The use according to any one of claims 1 to 6, wherein the plant is a monocotyledonous plant.

9. The use according to any one of claims 1 to 6, wherein the plant is selected from plant species belongs to the Poaceae family, the Solanaceae family, the Brassicaceae family, the Cucurbitaceae family, the Vitaceae family, the Rutaceae family, the Rosacea family, the Leguminosae family, the Nelumbonaceae family, the Pedaliaceae family, the Chenopodiaceae family, the Palmae family, the Musaceae family, the Malvaceae family, the Myrtaceae family, and the Capparidaceae family.

10. The use according to any one of claims 1 to 6, wherein the plant is selected from rice, maize, sorghum, wheat, barley, rye, Japanese millet, foxtail millet, tomato, eggplant, paprika, green pepper, potato, tobacco, Arabidopsis thaliana, rapeseed, shepherd's purse, Japanese white radish, cabbage, red cabbage, Brussels sprout (Petit vert), Chinese cabbage, bok-choy, kale, watercress, Japanese mustard spinach, broccoli, cauliflower, turnip, horseradish, mustard, cucumber, bitter gourd, pumpkin, melon, watermelon, grapes, lemons, oranges, navel oranges, grapefruit, mandarin, lime, sudachi, yuzu, Shiikuwasha, Tankan, apple, cherry, Japanese apricot, peach, loquat, apricot, plum, prunes, almonds, Japanese pear, pear, strawberry, raspberry, blackberry, black currant, cranberry, blueberry, soy, kidney beans, peas, fava beans, green soy beans, mung bean, chickpea, lotus (lotus root), sesame, spinach, beet, sugar beet, quinoa, hiyu, amaranthus, cockscomb, date palm, oil palm, coconut, acai, banana, Japanese banana, Manila hemp, cotton, okra, eucalyptus, Cleome gynandra, and Cleome spinosa.

11. The use according to claim 3, comprising cultivating a plant with symbiotic microorganisms at a sodium chloride concentration of 1.5 % by mass or more, wherein a survival rate of the plant is at least 10 %.

12. The use according to any one of claims 1 to 11, wherein the plant can grow under environment of the sodium concentration at which only 10 to 50% of the plants prior to the improvement of salt tolerance can grow.

## Patentansprüche

1. Verwendung von PERK13 (Prolin-reiche Extensin-ähnliche Rezeptorkinase 13) in einer Pflanze zur Verbesserung der Salztoleranz der Pflanze durch Unterdrückung oder Hemmung einer Funktion von PERK13 in der Pflanze,
wobei die Funktion von PERK13 so unterdrückt oder gehemmt wird, dass, wenn die Pflanze unter den Bedingungen einer Natriumchloridkonzentration von 1,0 Masse-% für 6 bis 24 Stunden hydroponisch kultiviert wird, die Menge an Natriumchlorid in ihrer Wurzel 90% oder weniger beträgt, bezogen auf die Menge (100%) an Natriumchlorid in der Wurzel, wenn die Pflanze unter den gleichen Bedingungen vor der Unterdrückung oder Hemmung der Funktion von PERK13 kultiviert wird.

2. Verwendung gemäß Anspruch 1, wobei die Unterdrückung oder Hemmung der Funktion von PERK13 durch Unterdrückung oder Hemmung der Expression des PERK13-Gens oder des PERK13-Proteins erfolgt.

3. Verwendung gemäß Anspruch 1 oder 2, wobei die Unterdrückung oder Hemmung der Funktion von PERK13 durch Einführen einer Mutation in die Pflanze erfolgt, um die Funktion ihres PERK13-Gens zu verringern oder zu stören.

4. Verwendung gemäß einem der Ansprüche 1 bis 3, wobei die Pflanze hinsichtlich einer Funktion mindestens eines Proteins, ausgewählt aus einem nicht-selektiven Kationenkanal, einem Na⁺/H⁺-Antiporter der Plasmamembran, einem vakuolären Na⁺/H⁺-Antiporter und einem Kaliumtransporter mit hoher Affinität, verbessert ist.

5. Verwendung gemäß einem der Ansprüche 1 bis 3, wobei die Pflanze eine Überexpression eines Gens aufweist, um mindestens ein Protein, ausgewählt aus einem nicht-selektiven Kationenkanal, einem Na⁺/H⁺-Antiporter der Plasmamembran, einem vakuolären Na⁺/H⁺-Antiporter und einem Kaliumtransporter mit hoher Affinität, zu produzieren.

6. Verwendung gemäß einem der Ansprüche 1 bis 3, wobei die Pflanze ein Transformant ist, in den ein Fremdgen eingeführt wurde,
wobei das Fremdgen mindestens eines der Gene, ausgewählt aus dem SOS1-Gen, dem SOS2-Gen, dem SOS3-Gen, dem NHX1-Gen und dem HKT1-Gen, ist.

7. Verwendung gemäß einem der Ansprüche 1 bis 6, wobei die Pflanze eine zweikeimblättrige Pflanze ist.

8. Verwendung gemäß einem der Ansprüche 1 bis 6, wobei die Pflanze eine einkeimblättrigePflanze ist.

9. Verwendung gemäß einem der Ansprüche 1 bis 6, wobei die Pflanze aus Pflanzenarten ausgewählt ist, die zur Familie der Poaceae, der Familie der Solanaceae, der Familie der Brassicaceae, der Familie der Cucurbitaceae, der Familie der Vitaceae, der Familie der Rutaceae, der Familie der Rosacea, der Familie der Leguminosae, der Familie der Nelumbonaceae, der Familie der Pedaliaceae, der Familie der Chenopodiaceae, der Familie der Palmae, der Familie der Musaceae, der Familie der Malvaceae, der Familie der Myrtaceae und der Familie der Capparidaceae gehören.

10. Verwendung gemäß einem der Ansprüche 1 bis 6, wobei die Pflanze ausgewählt ist aus Reis, Mais, Sorghum, Weizen, Gerste, Roggen, japanischer Hirse, Fuchsschwanzhirse, Tomate, Aubergine, Paprika, grünem Pfeffer, Kartoffel, Tabak, Arabidopsis thaliana, Raps, Hirtentäschelkraut, japanischem Rettich, Kohl, Rotkohl, Rosenkohl (Petit vert), Chinakohl, Pak Choy, Grünkohl, Brunnenkresse, japanischem Senfspinat, Brokkoli, Blumenkohl, Rübe, Meerrettich, Senf, Gurke, Bittermelone, Kürbis, Melone, Wassermelone, Trauben, Zitronen, Orangen, Navel-Orangen, Grapefruit, Mandarine, Limette, Sudachi, Yuzu, Shiikuwasha, Tankan, Apfel, Kirsche, japanischer Aprikose, Pfirsich, Mispel, Aprikose, Pflaume, Trockenpflaumen, Mandeln, japanischer Birne, Birne, Erdbeere, Himbeere, Brombeere, schwarzer Johannisbeere, Cranberry, Blaubeere, Soja, Kidneybohnen, Erbsen, Ackerbohnen, grünen Sojabohnen, Mungbohnen, Kichererbsen, Lotus (Lotuswurzel), Sesam, Spinat, Roter Bete, Zuckerrüben, Quinoa, Hiyu, Amarant, Hahnenkamm, Dattelpalme, Ölpalme, Kokosnuss, Acai, Banane, japanische Banane, Manilahanf, Baumwolle, Okra, Eukalyptus, Cleome gynandra und Cleome spinosa.

11. Verwendung gemäß Anspruch 3, umfassend das Kultivieren einer Pflanze mit symbiotischen Mikroorganismen bei einer Natriumchloridkonzentration von 1,5 Masse- % oder mehr, wobei die Überlebensrate der Pflanze mindestens 10% beträgt.

12. Verwendung gemäß einem der Ansprüche 1 bis 11, wobei die Pflanze unter den Bedingungen einer Natriumkonzentration wachsen kann, bei der nur 10 bis 50 % der Pflanzen vor der Verbesserung der Salztoleranz wachsen können.

## Revendications

1. Utilisation de la PERK13 (Proline-rich Extensin-like Receptor Kinase 13) dans une plante pour améliorer l'halotolérance de la plante en supprimant ou en inhibant une fonction de la PERK13 dans la plante,
dans laquelle la fonction de la PERK13 est supprimée ou inhibée de telle sorte que, lorsque la plante est cultivée par voie hydroponique dans les conditions d'une concentration en chlorure de sodium de 1,0 % en masse pendant 6 à 24 heures, une quantité de chlorure de sodium dans sa racine est de 90 % ou moins, par rapport à une quantité (100 %) de chlorure de sodium dans la racine en cultivant la plante dans les mêmes conditions avant de supprimer ou d'inhiber la fonction de la PERK13.

2. Utilisation selon la revendication 1, dans laquelle la suppression ou l'inhibition de la fonction de la PERK13 est mise en œuvre en supprimant ou en inhibant l'expression du gène PERK13 ou de la protéine PERK13.

3. Utilisation selon la revendication 1 ou 2, dans laquelle la suppression ou l'inhibition de la fonction de la PERK13 est mise en œuvre en introduisant une mutation dans la plante afin de diminuer ou d'interrompre la fonction de son gène PERK13.

4. Utilisation selon l'une quelconque des revendications 1 à 3, dans laquelle la plante est améliorée en ce qui concerne une fonction d'au moins une protéine choisie parmi un canal cationique non sélectif, un antiport Na⁺/H⁺ de membrane plasmique, un antiport Na⁺/H⁺ vacuolaire et un transporteur de potassium à haute affinité.

5. Utilisation selon l'une quelconque des revendications 1 à 3, dans laquelle la plante présente une surexpression d'un gène afin de produire au moins une protéine choisie parmi un canal cationique non sélectif, un antiport Na⁺/H⁺ de membrane plasmique, un antiport Na⁺/H⁺ vacuolaire et un transporteur de potassium à haute affinité.

6. Utilisation selon l'une quelconque des revendications 1 à 3, dans laquelle la plante est un transformant dans lequel un gène étranger a été introduit,
le gène étranger étant au moins un des gènes choisis parmi le gène SOS1, le gène SOS2, le gène SOS3, le gène NHX1 et le gène HKT1.

7. Utilisation selon l'une quelconque des revendications 1 à 6, dans laquelle la plante est une plante dicotylédone.

8. Utilisation selon l'une quelconque des revendications 1 à 6, dans laquelle la plante est une plante monocotylédone.

9. Utilisation selon l'une quelconque des revendications 1 à 6, dans laquelle la plante est choisie parmi les espèces végétales appartenant à la famille des Poacées, à la famille des Solanacées, à la famille des Brassicacées, à la famille des Cucurbitacées, à la famille des Vitacées, à la famille des Rutacées, à la famille des Rosacées, à la famille des Légumineuses, à la famille des Nélumbonacées, à la famille des Pédaliacées, à la famille des Chénopodiacées, à la famille des Palmiers, à la famille des Musacées, à la famille des Malvacées, à la famille des Myrtacées et à la famille des Capparidacées.

10. Utilisation selon l'une quelconque des revendications 1 à 6, dans laquelle la plante est choisie parmi : riz, maïs, sorgho, blé, orge, seigle, pied-de-coq, millet d'Italie, tomate, aubergine, paprika, poivron vert, pomme de terre, tabac, arabette des dames, colza, bourse-à-pasteur, radis blanc japonais, chou, chou rouge, chou de Bruxelles, chou chinois, pak-choï, chou frisé, cresson de fontaine, épinard moutarde japonais, brocoli, chou-fleur, navet, raifort, moutarde, concombre, coloquinte, citrouille, melon, pastèque, raisins, citrons, oranges, oranges doubles, pomélo, mandarine, lime, sudachi, yuzu, Shiikuwasha, Tankan, pomme, cerise, abricot japonais, pêche, néflier du Japon, abricot, prune, pruneaux, amandes, poire de Chine, poire, fraise, framboise, mûre sauvage, cassis, canneberge, myrtille, soja, haricots réniformes, pois, fèveroles, graines de soja vertes, haricot velu, pois chiche, lotus (racine de lotus), sésame, épinard, bette, betterave, quinoa, hiyu, amarante, célosie, dattier, palmier à huile, noix de coco, açaï, banane, banane japonaise, chanvre de Manille, coton, okra, eucalyptus, Cleome gynandra et Cleome spinosa.

11. Utilisation selon la revendication 3, comportant de mettre en culture une plante avec des micro-organismes symbiotiques à une concentration de chlorure de sodium de 1,5 % en masse ou plus, dans laquelle un taux de survie de la plante est d'au moins 10 %.

12. Utilisation selon l'une quelconque des revendications 1 à 11, dans laquelle la plante peut pousser dans un environnement dont la concentration en sodium permettait à seulement 10 à 50 % des plantes avant l'amélioration de l'halotolérance de pousser.
